(19) Europäisches Patentamt
European Patent Office
Office européen des brevets

(11) Veröffentlichungsnummer: **0 003 556**
**B1**

(12) # EUROPÄISCHE PATENTSCHRIFT

(45) Veröffentlichungstag der Patentschrift: **25.11.81**

(51) Int. Cl.³: **C 07 D 209/08**

(21) Anmeldenummer: **79100282.7**

(22) Anmeldetag: **31.01.79**

(54) **Verfahren zur Herstellung von Indoleninen.**

(30) Priorität: **10.02.78 DE 2805620**

(43) Veröffentlichungstag der Anmeldung: **22.08.79 Patentblatt 79/17**

(45) Bekanntmachung des Hinweises auf die Patenterteilung: **25.11.81 Patentblatt 81/47**

(84) Benannte Vertragsstaaten: **BE CH DE FR GB IT**

(56) Entgegenhaltungen:
**THE CHEMISTRY OF HETEROCYCLIC COMPOUNDS; Wiley Interscience, '72, »Indoles, Part One«, *Seiten 318, 319***
**TETRAHEDRON, Vol. 24, Nr. 5, März 1968, *Seiten 2227—2239***

(73) Patentinhaber: **BASF Aktiengesellschaft, Carl-Bosch-Strasse 38, D-6700 Ludwigshafen (DE)**

(72) Erfinder: **Opgenorth, Hans-Joachim, Dr. Chem, Hanns-Fay-Strasse 1, D-6710 Frankenthal (DE)**
Erfinder: **Scheuermann, Horst, Dr. Chem, Bexbacher Strasse 41, D-6700 Ludwigshafen (DE)**
Erfinder: **Laas, Harald, Dr. Chem, Woehlerstrasse 14, D-6701 Maxdorf 2 (DE)**
Erfinder: **Nissen, Axel, Dr. Chem, Panoramastrasse 51, D-6906 Leimen (DE)**

EP 0 003 556 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Verfahren zur Herstellung von Indoleninen

Die Erfindung betrifft ein Verfahren zur Herstellung von Verbindungen der Formel I

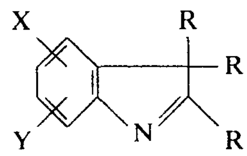

(I)

in der unabhängig voneinander

- R Alkyl oder Benzyl,
- X Wasserstoff, Chlor, Cyan, Brom, $C_1$- bis $C_4$-Alkyl, $C_1$- bis $C_4$-Alkoxy, $C_1$- bis $C_4$-Alkoxycarbonyl, Nitro oder $C_1$- bis $C_4$-Alkylsulfonyl und
- Y Wasserstoff, Chlor, Brom oder Methyl

bedeuten, das dadurch gekennzeichnet ist, daß man Verbindungen der Formel II

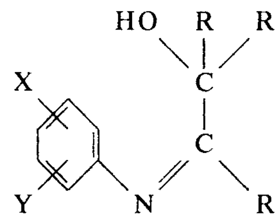

(II)

thermisch zu Verbindungen der Formel (III)

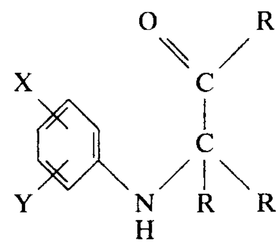

(III)

umlagert, die dann in Gegenwart eines sauren Katalysators zu einer Verbindung der Formel I cyclisiert werden.

Besonders bevorzugt ist ein Verfahren, bei dem man die Verbindungen der Formel II, d. h. die Schiffschen Basen aus Verbindungen der Formel IV

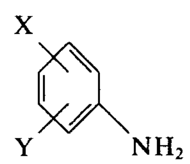

(IV)

und Verbindungen der Formel (V)

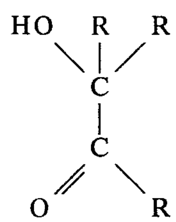

(V)

durch Kondensation in Gegenwart eines ein Azetrop bildenden Lösungsmittels und eines sauren Katalysators herstellt, danach den Katalysator entfernt oder inaktiviert, das Lösungsmittel und gegebenenfalls nicht umgesetzte Ausgangsmaterialien weitgehend abdestilliert und anschließend den Rückstand der thermischen Umlagerung und dem Ringschluß unterwirft.

Die Alkylreste R haben insbesondere 1 bis 4 C-Atome, bevorzugt ist Methyl.

Azeotrope bildende Lösungsmittel für die Umsetzung der Verbindungen der Formel IV und V sind beispielsweise Kohlenwasserstoffe und Chlorkohlenwasserstoffe wie Benzol, Cyclohexan, Methylcyclohexan, Chlorbenzol und vorzugsweise Toluol und Xylole.

Unter sauren Katalysatoren für die Herstellung der Verbindungen der Formel II werden erfindungsgemäß Verbindungen verstanden, die die Bildung der Verbindungen der Formel II katalysieren, jedoch danach entfernt oder inaktiviert werden können. Bevorzugt sind flüchtige Katalysatoren, die zusammen mit dem ein Azeotrop bildenden Lösungsmittel abdestilliert werden können, beispielsweise seien Ameisensäure, Chloressigsäure, Buttersäure und insbesondere Essigsäure und Propionsäure genannt. Weiterhin kommen als Katalysatoren Verbindungen in Betracht, die z. B. durch Amidbildung mit den Aminen der Formel IV inaktiviert werden können. Schließlich seien als Katalysatoren saure Ionenaustauscher genannt, die man anschließend abfiltrieren kann.

Für die Cyclisierung der Verbindungen der Formel III zu den Verbindungen der Formel I kommen als Katalysatoren Lewis-Säuren, wie Zinkchlorid, Aluminiumchlorid oder Bortrifluorid, organische Säuren wie Essigsäure, Propionsäure, Chloressigsäure, Benzoesäure, Stearinsäure, Benzol- oder Toluolsulfonsäure, Bernsteinsäure, Glutarsäure, Adipinsäure, saure Salze wie Natriumhydrogensulfat, Natriumdihydrogenphosphat sowie Mineralsäuren, wie phosphorige Säure und Phosphorsäure und vorzugsweise Salzsäure und Schwefelsäure in Betracht. Als besonders vorteilhaft hat es sich erwiesen, diese Katalysatoren in Form ihrer Salze mit den Aminen der Formel IV einzusetzen.

Die Umsetzung der Verbindungen der Formel IV und V wird zweckmäßigerweise so durchgeführt, daß man einen Aminüberschuß bis zu einem Molverhältnis von Amin zu Hydroxyketon von ~2 : 1 verwendet und die Komponenten (Verbindung IV, V, Katalysator und Lösungsmittel) unter azeotroper Entfernung des Wassers zum Sieden unter Rückfluß erhitzt. Das Lösungsmittel wird in der Regel in der 1- bis 5fachen Gewichtsmenge, bezogen auf Hydroxyketon, der Katalysator in ungefähr 0,1- bis 10%iger Menge, ebenfalls bezogen auf Hydroxyketon, verwendet.

Nach der Herstellung der Schiffschen Basen werden bei der bevorzugten Verwendung der flüchtigen Katalysatoren diese und das Lösungsmittel sowie der größte Teil des überschüssigen Amins der Formel IV abdestilliert. Anschließend wird so lange weiter erhitzt, bis die z. B. gaschromatographisch verfolgbare Umwandlung von II → III abgeschlossen ist. Der Temperaturbereich der Umwandlung liegt in der Regel zwischen 150 und 300° C, vorzugsweise 200 bis 250° C.

Die Cyclisierung zu den Verbindungen der Formel I wird danach zweckmäßigerweise im Temperaturbereich von 100 bis 250° C mit den vorzugsweise genannten Katalysatoren zwischen 140 und 200° C vorgenommen. Die benötigte Katalysatormenge liegt zwischen 0,1 und 20 Gewichtsprozent, vorzugsweise 1 und 10 Gewichtsprozent, bezogen auf eingesetztes Hydroxyketon.

Bei Anwesenheit von Aminen der Formel IV erfolgt die Cyclisierung besonders leicht. Zweckmäßigerweise wird daher der Aminüberschuß nach der Umsetzung zur Schiff-Base II nur so weit entfernt, daß mindestens die der verwendeten Katalysatormenge äquivalente Menge Amin zurückbleibt. Ein Überschuß von Amin zu Katalysator ist jedoch keineswegs nachteilig, sondern wirkt vielmehr reaktionsbeschleunigend. Da z. B. jedoch die destillative Trennung des Amins IV vom Indolenin I schwieriger ist als von den Verbindungen der Formel II und III, ist es vorteilhaft, den Aminüberschuß gering zu halten.

Einzelheiten der Reaktionsführung können den Beispielen entnommen werden, in denen sich Angaben über Teile und Prozente, sofern nicht anders vermerkt, auf das Gewicht beziehen.

Die Verbindungen der Formel I sind wichtige Vorprodukte zur Herstellung von Farbstoffen, insbesondere nach der Alkylierung des Stickstoffs und Deprotonierung zu den Verbindungen der Formel VI

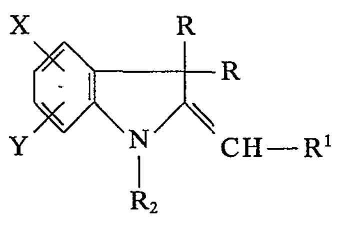

(VI)

in der $R^1 = R$ vermindert um eine $CH_2$-Gruppe ist und $R^2$ Alkyl mit 1 bis 4 C-Atomen, Hydroxyäthyl, Hydroxypropyl oder Benzyl bedeutet.

Die erfindungsgemäß herstellbaren Verbindungen der Formel I sind unmittelbar, d. h. ohne weitere Reinigung alkylierbar.

Aus der DE-OS 2 514 759 ist schon ein Verfahren zur Herstellung von Verbindungen der Formel I bekannt, bei dem jedoch bei allen Reaktionsschritten der für die Bildung der Schiffschen Base verwendete Katalysator anwesend ist. Damit sind bei der technischen Durchführung wesentliche Nachteile verbunden, die bei dem erfindungsgemäßen Verfahren vermieden werden:

1. Durch das im Reaktionsgemisch bei der Cyclisierung zu den Verbindungen der Formel I entstehende Wasser tritt Rückspaltung der Schiffschen Basen ein, was vor allem bei größeren Ansätzen zu Ausbeuteverlusten führt, wenn keine aufwendigen Vorsichtsmaßnahmen getroffen werden. Bei dem erfindungsgemäßen Verfahren ist diese Gefahr nicht gegeben, da die Schiff-Base II zunächst in die nicht mehr hydrolyseempfindliche Verbindung III überführt wird.
2. Die destillative Abtrennung eines Überschusses von mehr als 1 Mol Anilin pro Mol Indolenin erfordert wegen des geringen Siedepunktunterschieds eine wirksame Kolonne, da die Destillation aufgrund der leichten Zersetzlichkeit des Indolenins bei höheren Temperaturen zweckmäßigerweise im Vakuum vorgenommen werden muß. Dagegen kann das überschüssige Amin bei dem erfindungsgemäßen Verfahren von den höhersiedenden Verbindungen II bzw. III bei Normaldruck abdestilliert werden, so daß der Einsatz einer Kolonne nicht erforderlich ist.

## Beispiel 1

408 Teile 2-Methyl-2-hydroxy-butan-3-on, 558 Teile Anilin und 20 Teile Essigsäure werden in 650 Teilen Toluol so lange unter Auskreisen des Wassers erhitzt, bis kein Wasser mehr abgeschieden wird. Dann destilliert man die flüchtigen Anteile ab, bis die Innentemperatur 220° C erreicht hat. Das Destillat kann ohne weitere Behandlung in einem folgenden Ansatz wieder verwendet werden. Man hält die Temperatur dann 5 Stunden bei 220° C, kühlt danach auf 160° C ab und gibt 40 Teile konz. Salzsäure zu. Nach weiterem dreistündigem Erhitzen, wobei das gebildete Wasser abdestilliert, ist die Umsetzung vollständig. Das Produkt wird entweder direkt weiterverarbeitet oder destilliert. Man erhält 498 Teile 2,3,3-Trimethyl-indolenin entsprechend 78,5% der Theorie, bezogen auf 2-Methyl-2-hydroxy-butan-3-on.

## Beispiel 2

Man verfährt wie in Beispiel 1, setzt jedoch statt 40 Teile Salzsäure 40 Teile konz. Schwefelsäure zu. Man erhält 473 Teile 2,3,3-Trimethyl-indolenin entsprechend 74,4% der Theorie, bezogen auf 2-Methyl-2-hydroxy-butan-3-on.

## Beispiel 3

Man verfährt wie in Beispiel 1, es wird jedoch nach dem fünfstündigen Erhitzen im Vakuum destilliert. Man erhält 576 Teile 2-Anilino-2-methyl-butan-3-on (Schmelzpunkt 65 bis 66° C) entsprechend 81,4% der Theorie, bezogen auf 2-Methyl-2-hydroxy-butan-3-on.

## Beispiel 4

246 Teile p-Anisidin, 204 Teile 2-Methyl-2-hydroxy-butan-3-on und 10 Teile Essigsäure werden in 650 Teilen Toluol so lange unter Entfernung des Wassers erhitzt, bis kein Wasser mehr abgeschieden wird. Dann destilliert man das Lösungsmittel ab und erhitzt 5 Stunden auf 220° C. Nach dem Abkühlen auf 160° C werden 10 Teile konz. Schwefelsäure zugegeben; es wird diese Temperatur 3 Stunden gehalten, wobei das gebildete Wasser abdestilliert wird. Durch Destillation im Vakuum bei 21 mbar erhält man 318,7 Teile 5-Methoxy-2,3,3-trimethyl-indolenin (Schmelzpunkt 53 bis 54° C) entsprechend 84,3% der Theorie.

Auf entsprechende Weise erhält man folgende Indoleninderivate:

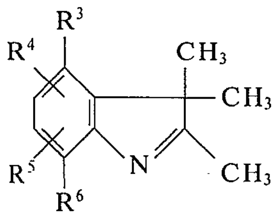

| Beispiel | $R^3$ | $R^4$ | $R^5$ | $R^6$ | Ausbeute |
|---|---|---|---|---|---|
| 5 | H | Cl | H | H | 75,6 |
| 6 | H | H | Cl | $CH_3$ | 71,4 |
| 7 | H | $CH_3$ | H | H | 77,4 |
| 8 | H | Br | H | H | 73,9 |
| 9 | H | H | H | CN | 55,6 |
| 10 | H | $SO_2CH_3$ | H | H | 64,6 |
| 11 | H | $OC_2H_5$ | H | H | 72,3 |
| 12 | H | $OC_4H_9$ | H | H | 69,4 |

**Patentansprüche**

1. Verfahren zur Herstellung von Indoleninen der allgemeinen Formel

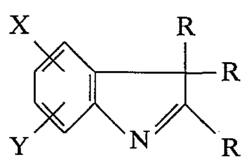

in der unabhängig voneinander

R Alkyl oder Benzyl,
X Wasserstoff, Chlor, Brom, Cyan, $C_1$- bis $C_4$-Alkyl, $C_1$- bis $C_4$-Alkoxy, $C_1$- bis $C_4$-Alkoxycarbonyl, Nitro oder $C_1$- bis $C_4$-Alkylsulfonyl und
Y Wasserstoff, Chlor, Brom oder Methyl

bedeuten, dadurch gekennzeichnet, daß man Verbindungen der Formel

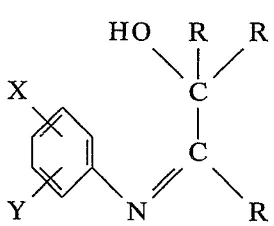

(II)

thermisch zu Verbindungen der Formel (III)

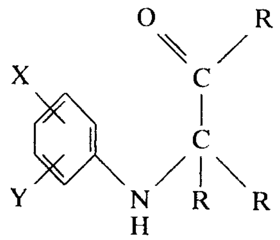

(III)

umlagert, die dann in Gegenwart eines sauren Katalysators zu einer Verbindung der Formel I cyclisiert werden.

2. Verfahren gemäß Anspruch 1, dadurch gekennzeichnet, daß man für die Cyclisierung der Verbindungen III als saure Katalysatoren Salzsäure oder Schwefelsäure verwendet.

3. Verfahren gemäß Anspruch 1, dadurch gekennzeichnet, daß man die Verbindungen der Formel II aus Verbindungen der Formel IV

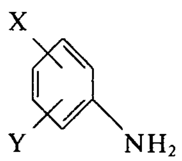

(IV)

und Verbindungen der Formel (V)

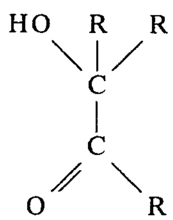

(V)

durch Kondensation in Gegenwart eines ein Azeotrop bildenden Lösungsmittels und eines sauren Katalysators herstellt, danach den Katalysator entfernt oder inaktiviert, das Lösungsmittel und gegebenenfalls nicht umgesetzte Ausgangsmaterialien weitgehend abdestilliert und anschließend den Rückstand der thermischen Umlagerung und dem Ringschluß unterwirft.

4. Verfahren gemäß Anspruch 3, dadurch gekennzeichnet, daß man für die Bildung der Schiff-Base als saure Katalysatoren eine flüchtige Carbonsäure verwendet.

**Claims**

1. A process for the preparation of an indolenine of the general formula

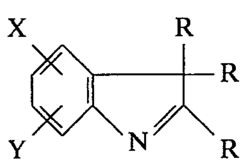

where, independently of one another,

R is alkyl or benzyl,
X is hydrogen, chlorine, bromine, cyano, $C_1-C_4$-alkyl, $C_1-C_4$-alkoxy, $C_1-C_4$-alkoxycarbonyl, nitro or $C_1-C_4$-alkylsulfonyl and
Y is hydrogen, chlorine, bromine or methyl,

characterized in that a compound of the formula

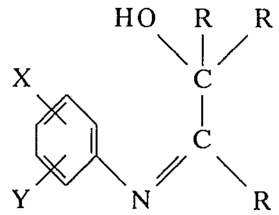

(II)

is thermally rearranged to give a compound of the formula (III)

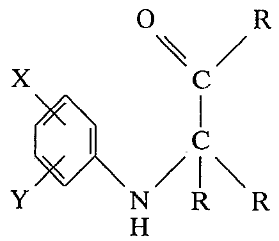

(III)

which is then cyclized in the presence of an acid catalyst to give a compound of the formula I.

2. A process as claimed in claim 1, characterized in that hydrochloric acid or sulfuric acid is used as the acid catalyst for cyclizing the compounds III.

3. A process as claimed in claim 1, characterized in that a compound of the formula II is prepared from a compound of the formula IV

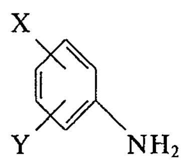

(IV)

and a compound of the formula (V)

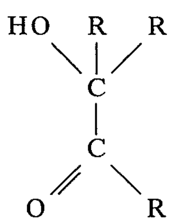

(V)

by condensation in the presence of an azeotrope-forming solvent and of an aid catalyst, the catalyst is then removed or inactivated, the solvent and any unconverted starting materials are substantially distilled off and the residue is then subjected to thermal rearrangement and cyclization.

4. A process as claimed in claim 3, characterized in that a volatile carboxylic acid is used as the acid catalyst for the formation of the Schiff base.

**Revendications**

1. Procédé de préparation d'indolénines de formule générale

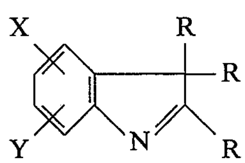

dans laquelle les symboles utilisés représentent, indépendamment les uns des autres,

R un groupe alkyle ou benzyle,
X l'hydrogène, le chlore, le brome, un groupe cyano, alkyle en C 1—C 4, alcoxy en C 1—C 4, (alcoxy en C 1—C 4)-carbonyle, nitro ou (alkyle en C 1—C 4)-sulfonyle et
Y représente l'hydrogène, le chlore, le brome ou un groupe méthyle,

caractérisé en ce que l'on transpose des composés de formule

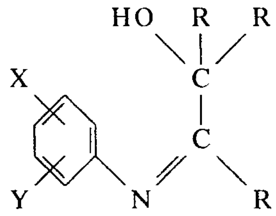

(II)

à la chaleur, en composés de formule (III)

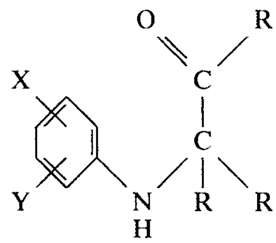

(III)

qu'on cyclise ensuite en un composé de formule I en présence d'un catalyseur acide.

2. Procédé selon la revendication 1, caractérisé en ce que le catalyseur acide utilisé pour la cyclisation des composés III est l'acide chlorhydrique ou l'acide sulfurique.

3. Procédé selon la revendication 1, caractérisé en ce que l'on prépare les composés de formule II à partir de composés de formule IV

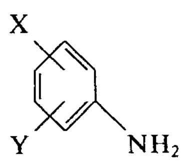

(IV)

et de composés de formule (V)

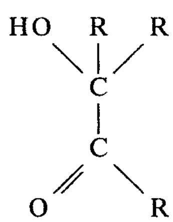

(V)

par condensation en présence d'un solvant formant un azéotrope et d'un catalyseur acide, en faisant suivre de l'élimination ou de la désactivation du catalyseur, de l'élimination pratiquement complète par distillation du solvant et le cas échéant des produits de départ non convertis, après quoi on soumet le résidu à la transposition à la chaleur et à la cylisation.

4. Procédé selon la revendication 3, caractérisé en ce que l'on utilise un acide carboxylique volatil en tant que catalyseur acide pour la formation de la base de Schiff.